(19) Europäisches Patentamt / European Patent Office / Office européen des brevets

(11) **EP 4 434 579 A1**

(12) **EUROPEAN PATENT APPLICATION**

(43) Date of publication:
**25.09.2024 Bulletin 2024/39**

(21) Application number: **23382269.1**

(22) Date of filing: **23.03.2023**

(51) International Patent Classification (IPC):
**A61P 5/48** (2006.01)   **A61K 38/25** (2006.01)
**C07K 14/60** (2006.01)

(52) Cooperative Patent Classification (CPC):
**A61K 38/25; A61P 5/48; C07K 14/4702;**
**C07K 14/60; C12N 9/12; C12Y 207/11024**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Servizo Galego de Saude**
  **15703 Santiago de Compostela (ES)**
- **Fundación Instituto de Investigación Sanitaria de Santiago de Compostela (FIDIS)**
  **15706 Santiago de Compostela (ES)**
- **Universidade de Santiago de Compostela**
  **15782 Santiago de Compostela (ES)**

(72) Inventors:
- **PAZOS RANDULFE, Yolanda**
  **15706 Santiago de Compostela (ES)**
- **PÉREZ CAMIÑA, Jesús**
  **15706 Santiago de Compostela (ES)**

- **DOMÍNGUEZ MUÑOZ, Juan Enrique**
  **15706 Santiago de Compostela (ES)**
- **CID DÍAZ, Tania**
  **15706 Santiago de Compostela (ES)**
- **SANTOS ZÁS, Icía**
  **15706 Santiago de Compostela (ES)**
- **LEAL LÓPEZ, Saúl**
  **15706 Santiago de Compostela (ES)**
- **CALVIÑO LODEIRO, Andrea**
  **15706 Santiago de Compostela (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

Remarks:
The complete document including Reference Table(s) and the Sequence Listing(s) can be downloaded from the EPO website

(54) **OBESTATIN FOR USE AS ANTINEOPLASTIC AGENT IN THE TREATMENT OF PANCREATIC CANCER**

(57) Obestatin for use as antineoplastic agent in the treatment of pancreatic cancer. The present invention refers to the use obestatin as antineoplastic agent in the treatment of pancreatic cancer.

EP 4 434 579 A1

## Description

### FILED OF THE INVENTION

The present invention refers to the medical field. Particularly, the present invention refers to the use of obestatin as antineoplastic agent in the treatment of pancreatic cancer.

### STATE OF THE ART

### Obestatin

Obestatin is a 23-amino-acid peptide derived from the pre-proghrelin precursor. This novel peptide was first isolated in 2005. Initially, obestatin was described as a circulating peptide that has a pulsatile secretion with an ultradian rhythm similar to ghrelin. Obestatin was reported as a molecule with an action opposite to the orexigenic action of ghrelin; however, its role in the food intake is still very controversial.

Preproghrelin is a polypeptide encoded by the GHRL gene. After translation, the signal peptide is removed and processed into both ghrelin and obestatin. The post-transcriptional processing of pre-proghrelin generates different molecules derived from ghrelin based on splicing and post-transcriptional residues modification.

Preproghrelin is composed of a signal peptide and a fragment of 94 amino acids: the proghrelin. In the endoplasmic reticulum, the signal peptide is removed and proghrelin suffers from several proteolytic processes. The proghrelin produces mature ghrelin and c-ghrelin, a 66 amino acid peptide. C-ghrelin can freely circulate and/or be processed resulting in smaller peptides including obestatin.

After excision of the obestatin precursor, a lysine residue is removed from the carboxy-terminal end, while the anterior glycine residue donates an amide group to the terminal carboxy end leucine residue. This amidation at the carboxy terminal end appears to be a key modification necessary for the bioactivity of obestatin.

An obestatin truncated fragment of 13 amino acids has been identified in stomach extracts: obestatin-(11-23). Whether (11-23)-obestatin is formed physiologically and the mechanisms by which it is formed are still largely unknown. Several authors suggest that (1 1-23)-obestatin may have a biological activity analogous to obestatin [Begoña O. Alén et al. The NMR structure of human obestatin in membrane-like environments: insights into the structure-bioactivity relationship of obestatin. PLoS One. 2012; 7(10):e45434].

Although in humans the main obestatin-producing cells are found in the stomach and the rest of the gastrointestinal tract, obestatin has also been found in other tissues. In addition to the stomach, reactive cells for obestatin have also been found in the periphery of the islets of the pancreas, as well as in breast glands. In other tissues such as the pituitary gland, prostate, testicles, placenta, ovaries or thyroid, a weak expression of obestatin can be detected.

The orphan G protein-coupled receptor 39 (GPR39) has initially been reported to be the receptor of obestatin, however, some authors questioned any stimulatory activity of obestatin on GPR39 suggested that obestatin does not activate GPR39 and that its ligand is yet to be discovered.

Other receptors such as glucagon-like peptide-1 receptor (GLP1R) have been proposed as mediators of the action of obestatin, however this fact has been questioned due to the inability of obestatin to displace GLP1 in HEK293T cells overexpressing GLP1R.

Zinc ions have been suggested likewise as a specific ligand of the GPR39 receptor, considering it as a key component of the Zn metabotropic signalling.

Obestatin has been shown to have a variety of iodination levels. Peptides with different levels of iodination have distinct metabolizing properties and presumably different biological activities. In this sense, it has been demonstrated that mono-iodo-obestatin is able to bind specifically to the GPR39 receptor in transiently transfected HEK293T cells with plasmids encoding human and murine GPR39.

There is a great deal of controversy about whether obestatin is the ligand of the GPR39 receptor, however, different studies support this fact. Obestatin is able to induce the expression of transcription factors related to gastrointestinal activity such as c-fos in wild-type mice, while this effect is mitigated when using knockout mice for the GPR39 receptor. In addition, the signal triggered by the obestatin is inhibited by GPR39 receptor silencing. Coimmunoprecipitation assays conclusively prove the binding of obestatin as a ligand of the GPR39 receptor.

A wide range of tissues expresses GPR39, both in humans and rodents. The expression of GPR39 has been tested in different tissues through the analysis of protein and RNA expression. Despite the fact that obestatin was originally described as an opponent of ghrelin activity this effect is still very controversial.

Several studies still support the involvement of the obestatin/GPR39 system in the regulation of energy homeostasis and intraperitoneal administration of obestatin in rodents has been shown to reduce food intake. Obestatin also appears to be involved in thirst inhibition, anxiety reduction, sleep regulation and cortical neuron aggregation. It is also able to decrease apoptosis in adipose tissue, inflammation and insulin resistance.

The obestatin/GPR39 system has also been proposed as an important regulator of adipocyte metabolism and adipogenesis. In this sense, preproghrelin and consequently obestatin are overexpressed during adipogenesis *in vitro.* In addition, studies in which obestatin is removed or blocked demonstrated the importance of this peptide during adipogenesis. In human adipose tissue, decreased GPR39 expression was found in patients with obesity-associated type 2 diabetes mellitus.

It has been demonstrated that the obestatin/GPR39 system plays a role in the myogenesis regulation. Preproghrelin is increased during L6E9 cell differentiation. Overexpression of the obestatin/GPR39 system in skeletal muscle promotes stimulation of expansion and differentiation of satellite cells and hypertrophy of the myofiber, increasing regenerative capacity after muscle damage.

On the other hand, in cardiac muscle, obestatin, through its receptor, is capable of increase the secretion of epinephrine from the sympathetic neurons, which leads to an increase in the ex vivo and in vivo contraction force.

GPR39 was found upregulated in a hippocampal cell line resistant against cell death stimulators. The overexpression of GPR39 in these cells is able to protect against different proapoptotic signals such as oxidative stress and endoplasmic reticulum stress. These data suggest a possible role for GPR39 as a potential neuroprotective mediator target.

Obestatin has been also involved in the improvement of the peripheral nerve repair, by enhancing both neuroprotection and remyelination, and thus the regeneration of the damaged nerve, counteracting the severe loss of function and the atrophy of the innervated muscle.

**Pancreatic cancer**

The pancreas is central to the control of energy consumption and metabolism. This organ consists of two morphologically and functionally distinct components: the exocrine unit, which produces digestive enzymes, and the endocrine unit, represented by the islets of Langerhans. Insulin and glucagon are among the hormones secreted by the islets that play a key role in regulating blood glucose levels. Different diseases affect the exocrine and endocrine pancreas. Pancreatitis (acute and chronic) and pancreatic cancers, most of which are ductal carcinomas (PDAC, up to 93%), originate from the exocrine pancreas, while diabetes and rare pancreatic neuroendocrine tumours arise from the islets. Although pancreatic cancer does not have a high incidence, it does have a high mortality, reaching annual figures that overlap with incidence. Moreover, in the case of women it increases every year with values of 5.6/10E5 inhabitants per year, representing the second worst predicted cancer after lung cancer. These increases are especially important in countries such as France and Spain, where tobacco use became widespread among women in the 1970s and 1980s.

Pancreatic cancer is one of the most aggressive cancers with the worst prognosis, mainly due to the lack of specific symptoms in the early stages of development and the consequent late diagnosis. PDAC has a poor prognosis, with a life expectancy of only 5% at 5 years. Despite advances in the understanding of PDAC development and improvements in surgical techniques, the survival rate has not changed substantially since the introduction of pancreaticoduodenectomy 80 years ago. Currently, surgical resection is the only potentially curative treatment for early stage PDAC, but this only occurs in less than 20% of patients.

**Current approaches in pancreatic cancer management**

Patients with pancreatic cancer often present with non-specific symptoms leading to delayed presentation. The most common presenting symptoms include asthenia (86%), weight loss (85%), anorexia (83%), abdominal pain (79%), dark urine (59%), jaundice (56%), nausea (51%), back pain (49%), diarrhoea (44%), vomiting (33%), steatorrhea (25%), and thrombophlebitis (3%). The most frequent signs are jaundice (55%), hepatosplenomegaly (39%), right upper quadrant mass (15%), cachexia (13%), Courvoisier's sign (13%), epigastric mass (9%), and ascites (5%). Other rare presentations can be unexplained superficial thrombophlebitis (classic Trousseau's syndrome) and paraneoplastic skin manifestations such as cicatricial and bullous pemphigoid.

Pancreatic tumours are usually hypovascular and thus are better visualized under contrast imaging. Computerized tomography (CT) scan is the selected study and can provide an evaluation of the disease extent, which will determine the resectability of the tumour. Magnetic Resonance Imaging (MRI) is suitable in differentiate tumours from the normal surrounding pancreas or CP or in detecting liver and early lymph node metastasis. Endoscopic ultrasound (EUS) is essential in the diagnosis and management of pancreatic cancer, for measuring the depth of the tumour or to guide a fine needle aspiration biopsy for diagnosis without aggressive surgery. Since a majority of patients have pancreatic head/body tumours which can lead to biliary obstruction, endoscopic retrograde cholangiopancreatography (ERCP) is used to relieve biliary obstruction. If the patient has clearly resectable pancreatic cancer and is a good surgical candidate, it is reasonable to proceed to surgery without a tissue diagnosis since surgery is the only curative procedure for these patients. However, even in the patients with complete resection there are local recurrence rates of 35-60% and systemic recurrence rates as high as 80-90%. Positron emission tomography (PET)/CT scans have also been helpful in detecting metastatic disease prior to surgical resection.

Tumour markers are not useful in diagnosing pancreatic cancer. CA 19-9 is the most commonly used marker in pancreatic cancer, having a sensitivity ranging from 70% to 92% and specificity of 68-92%. However, CA 19-9 does have some role in prognosis and monitoring disease activity in patients with high initial levels. Other markers have been studied only in the preclinical setting, such as macrophage inhibitory cytokine 1 (MIC-1), osteopontin, tissue inhibitor of metalloproteinase 1, and hepatocarcinoma-intestine-pancreas protein.

Tumour-node metastasis (TNM) staging is the preferred staging system for pancreatic cancer. Conventional or pylorus-preserving Whipple operation (pancreaticoduodenectomy) with standard lymphadenectomy and distal pancreatectomy with splenectomy are the surgeries of choice for cancers of the head/neck and body/tail, respectively. Other important predictors of survival include tumor staging, status of the surgical margin, and node status at the time of surgery. In a patient with resected disease 5-year survival is about 20% and median survival is 18-24 months. Even in the patients with complete resection there are local recurrence rates of 35-60% and systemic recurrence rates as high as 80-90%. Pain occurs in 50-70% of pancreatic cancer patients. Narcotics are usually used for chronic pain treatment; however, celiac plexus neurolysis is used in selected patients. Patients with advanced disease often have nutritional issues. Certain approaches involving improved exocrine function of the pancreas by pancreatic enzyme replacement therapy, increase survival in patients with unresectable pancreatic cancer (Domínguez-Muñoz JE, et al. Impact of the treatment of pancreatic exocrine insufficiency on survival of patients with unresectable pancreatic cancer: a retrospective analysis. BMC Cancer. 2018;18(1):534. doi: 10.1186/s12885-018-4439-x).

Neoadjuvant chemotherapy is used to down stage the tumour and increase the potential for an R0 (margin negative) resection. Gemcitabine or 5-fluorouracil (5FU) chemotherapy, 5FU + oxaliplatin + irinotecan + leucovorin (FOLFIRINOX) or nab-paclitaxel are the drugs of choice.

Almost 30% of patients present locally advanced pancreatic cancer with a median survival of 8-12 months and a 5-year survival rate of approximately 6%. These tumours are considered inoperable and the treatments of choice are chemotherapy alone (gemcitabine, FOLFIRINOX, nab-paclitaxel) or in combination with radiotherapy in order to eradicate ocult micrometastasis and downstage the primary tumour. Recurrence is observed in up to 70% of receted patients [liver (47%), lung (22%), tumor bed/regional lymph nodes (17%), and peritoneum (13%)]; consequently, adjuvant chemotherapy is recommended in all resected cancers including T1N0 disease. The standard first line therapy for these patiens is gemcitabine although some improvements have been reached with nab-paclitaxel. As second line therapy, 5-FU, FOLFOX, or FOLFIRINOX are used. The chemotherapy toxicities in these aggressive managements of pancreatic cancer can also impact patient survival.

The refinement of molecular profiling, both germline and somatic, has improved the outcomes for pancreatic cancer and beneficial effects are seen with adjuvant chemotherapy. The NCCN Guidelines for Pancreatic Adenocarcinoma reflect these advances, and recommend germline testing for all patients with pancreatic cancer and the consideration of a molecular analysis for those with metastatic disease (Tempero MA. NCCN Guidelines Updates: Pancreatic Cancer. J Natl Compr Canc Netw. 2019 May 1;17(5.5):603-605. doi: 10.6004/jnccn.2019.5007).

In summary, survival in PDAC has not improved much in recent years, mainly due to the great intra- and inter-tumoral heterogeneity, which makes each tumour unique, representing a challenge for conventional therapies. Personalised medicine has brought improvements in survival in other types of cancer, such as breast, lung or melanoma; however, only recently molecular profiles have been implemented in patients with pancreatic cancer that could allow the identification of individuals with useful alterations.

In this scenario, the present invention is focused on solving the above cited problem and provides a new treatment aimed at inhibiting tumour growth, directly or indirectly, through the remodelling of parameters associated with the tumour microenvironment.

## DESCRIPTION OF THE INVENTION

### Brief description of the invention

The present invention refers to the use of obestatin for the treatment of pancreatic cancer, preferably to the use of obestatin as antineoplastic agent in the treatment of pancreatic cancer.

Such it is shown in **Example 2,** particularly in **Example 2.1,** obestatin inhibits mitogenic features (ERK1/2 activity) in pancreatic cancer cells both murine (KPC cells) and human (PANC1, RWP1 and BxPC3). Indeed, obestatin treatment does not activate the proliferation of different pancreatic cancer cell lines in chronic treatments.

On the other hand, such as it is shown in **Example 2,** particularly in **Example 2.2,** the in vivo syngeneic orthotopic murine pancreatic cancer model, in which KPC FC1242 cells were inoculated into the pancreas of C57BL/6 mice, was designed to evaluate the therapeutic efficacy of obestatin. Obestatin treatment mitigated the manifestations of cancer-associated cachexia while maintaining food intake. However, obestatin not only mitigated cachectic symptoms, but also inhibited tumour growth. The size and weight of tumours in the obestatin-treated group were significantly smaller than those in the control or vehicle-treated groups **(Example 2.2).**

Consistent with these results, obestatin treatment reduced the number of Ki67+ proliferating cells and inhibited effectors of the MAPK pathway (ERK1/2), while increasing signalling through PI3K-Akt **(Example 2.3).**

In addition, obestatin activated the caspase pathway (cleaved caspase 3) and apoptosis through Bax **Example 2.4.** Pro-apoptotic activity was associated with inhibition of autophagic flux through upregulation of p62. Obestatin increased the expression of HIF1a, a regulator of oxygen homeostasis, compared to control or vehicle-treated **Example 2.5.**

Notably, obestatin treatment inhibited the expression of fibrosis-related markers collagen and fibronectin **Example 2.6,** implying that this peptide shapes the tumour microenvironment towards conditions unfavourable for tumour growth through the regulation of fibrosis. Paradoxically, obestatin increased the expression of myofibroblastic cancer-associated fibroblasts (myCAF) markers, including aSMA, HSP47 and vimentin **Example 2.6.** Moreover, **Example 2.6** shows the augmentation of aSMA expression in tumor with obestatin treatment. Furthermore, obestatin treatment inhibited the NF-kB and JAK/STAT pathways, nodes involved in stromal modification and tumour growth through the determination of CAFs [myCAF/inflammatory-like CAF (iCAF) ratio], **Example 2.6.**

Although we cannot exclude that these effects are partly a consequence of a direct targeting of tumour cells, these results suggest that obestatin regulates CAF plasticity towards a myCAF phenotype that restricts rather than promotes tumour growth. Notably, obestatin treatment mitigated the manifestations of pancreatic cancer-associated cachexia, reducing the skeletal muscle weight loss caused by tumour **Example 2.7.**

In summary, obestatin in the presence of cancer cells allows remodelling of the tumour microenvironment by facilitating the reprogramming of key cells thereby inhibiting tumour proliferation and growth.

So, according to the results shown in **Example 2,** the first embodiment of the present invention refers to obestatin comprising or consisting of the amino acid sequence of Formula I:

$$C\text{-FNAP}\mathbf{X_1X_2X_3}\text{GIKLSG}\mathbf{X_4}\text{Q}\mathbf{X_5X_6X_7}\text{H}\mathbf{X_8X_9X_{10}}\text{L-}N \qquad \textbf{(I)}$$

or any fragment thereof comprising or consisting of the aminoacid sequence of Formula II:

$$C\text{-LSG}\mathbf{X_{4Q}X_5X_6X_7}\text{H}\mathbf{X_8X_9X_{10}}\text{L-}N \qquad \textbf{(II)}$$

or any molecule comprising the above defined obestatin, for use as antineoplastic agent in the treatment of pancreatic cancer, wherein: $X_1$ can be substituted by the amino acid F or C, $X_2$ can be substituted by the amino acid D or N, $X_3$ can be substituted by the amino acid V or I, $X_4$ can be substituted by the amino acid V, A or P, $X_5$ can be substituted by the amino acid Y or S, $X_6$ can be substituted by the amino acid Q, H, L or D, $X_7$ can be substituted by the amino acid Q or E, $X_8$ can be substituted by the amino acid S or G, $X_9$ can be substituted by the amino acid Q or R, and $X_{10}$ can be substituted by the amino acid A, T or P.

In a preferred embodiment, the amino acid sequence of Formula I is selected from the list comprising or consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 or SEQ ID NO: 9 or the amino acid sequence of Formula II is selected from the list comprising or consisting of: SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17 or SEQ ID NO: 18.

Obestatin and variants or derivatives thereof may be synthesized, for example, although without limitation, by chemical synthesis, recombinant DNA techniques, isolation of natural sources or by *in vitro* proteolysis. Obestatin may be produced recombinantly, not only directly but as a fusion polypeptide together with a heterologous polypeptide, which may contain, for example, although without limiting ourselves, a signal sequence, or another polypeptide which has a cut-off site for a protease, for example, although without limiting ourselves, at the N-terminal end of the mature protein or of the polypeptide.

In a preferred embodiment, the present invention refers to obestatin for use as antineoplastic agent. In a preferred embodiment, the treatment comprises the diminution of size pancreatic tumour. In a preferred embodiment, obestatin is administered systemically via subcutaneous devices (subcutaneous pumps) to the patient to be treated.

The second embodiment of the present invention refers to a pharmaceutical composition comprising or consisting of obestatin, or a fragment thereof, or any molecule comprising the above defined obestatin, dissolved in a suitable solvent and, optionally, pharmaceutically acceptable excipients or carrier, wherein obestatin comprises or consists of the amino acid sequence of Formula I:

$$C\text{-FNAP}\mathbf{X_1X_2X_3}\text{GIKLSG}\mathbf{X_4}\text{Q}\mathbf{X_5X_6X_7}\text{H}\mathbf{X_8X_9X_{10}}\text{L-}N \qquad \textbf{(I)}$$

and the fragment thereof comprising or consisting of the aminoacid sequence of Formula II:

$$C\text{-LSG}\mathbf{X_4}\text{Q}\mathbf{X_5X_6X_7}\text{H}\mathbf{X_8X_9X_{10}}\text{L-}N \qquad \textbf{(II)}$$

wherein: $X_1$ can be substituted by the amino acid F or C, $X_2$ can be substituted by the amino acid D or N, $X_3$ can be substituted by the amino acid V or I, $X_4$ can be substituted by the amino acid V, A or P, $X_5$ can be substituted by the amino acid Y or S, $X_6$ can be substituted by the amino acid Q, H, L or D, $X_7$ can be substituted by the amino acid Q or E, $X_8$ can be substituted by the amino acid S or G, $X_9$ can be substituted by the amino acid Q or R, and $X_{10}$ can be substituted by the amino acid A, T or P.

In a preferred embodiment, the amino acid sequence of Formula I is selected from the list comprising or consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 or SEQ ID NO: 9 or wherein the amino acid sequence of Formula II is selected from the list comprising or consisting of: SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17 or SEQ ID NO: 18.

In a preferred embodiment, the present invention refers to the above defined pharmaceutical composition for use in the treatment of pancreatic cancer, wherein the treatment comprises the reduction of tumour size. In a preferred embodiment, the composition is administered systemically to the patient. In a preferred embodiment, obestatin is prepared in aqueous medium.

The third embodiment of the present invention refers to a method for treating patients suffering from pancreatic cancer, which comprises treating the patient with a therapeutically effective dose or amount of obestatin or any molecule comprising thereof, or any fragment thereof, or any pharmaceutical composition comrpising obestatin of fragments thereof.

Preferably, the medicament comprises obestatin in a therapeutically effective quantity, understanding as "therapeutically effective quantity" the quantity of obestatin, which produces the desired effect. The dosage to obtain a therapeutically effective quantity depends on a variety of factors, such as, for example, the weight, sex or tolerance of the individual the medicament is going to be administered to.

Although, as cited above, obestatin or the pharmaceutical composition comprising obestatin is preferably administered systemically to the patient, obestatin can be also presented in parenteral preparations, either solid or liquid. In a preferred embodiment, the vehicle is the aqueous medium, however, in some circumstances, non-aqueous liposoluble vehicles (benzene, benzoate or vegetable oils) and water-miscible hydrophilic vehicles (for example ethanol, glycerine or polyethylene glycol) can be used to achieve preparations of obestatin with a longer effect, a higher solubility of the peptide and/or an increase in the stability of the peptide. Although surfactant excipients can be used to improve the solubility of obestatin by reducing the surface tension, which may have detergent, emulsifier, foaming and wetting power, in a preferred embodiment, the surfactant excipients are selected from the list: Polysorbate 80 Tween 80®, Polysorbate 20 Tween 20®, Lecithins, Solutol®, Pluronic® or Polyethylene glycol derivatives. Although pH regulating excipients can be used to approximate the pH of the preparation of obestatin to a physiological value and an increase in the stability of obestatin, in a preferred embodiment, the pH regulating excipients are selected from the list: Acetates, Arginine, Boric acid, Carbonates, Glycine, Lysine or Phosphates. Although isotonizing agents can be used to ensure the isotonicity of the preparation of obestatin, in a preferred embodiment, the isotonizing agents are selected from the list: Sodium Chloride, Potassium Chloride, Glucose, Sucrose, and Mannitol. Although antioxidants can be added to protect the active obestatin from oxidation, in a preferred embodiment, antioxidants are selected from the list: Derivatives of ascorbic acid, Salts of sulphurous acid, Monothioglycerol, Amino acids (cysteine, methionine), Monosodium glutamate or Glutathione. Although antimicrobial preservatives are used to prevent the growth of microorganisms in the preparation of obestatin, in a preferred embodiment, antimicrobial preservatives are selected from the list: Benzalkonium Chloride, Benzethonium Chloride, Coristor myristyl-gamma-picolinium, Benzyl Alcohol, Chlorobutanol, m-Cresol, Phenol, 2-Phenoxyethanol, Methyl Paraben, Propyl Paraben, Nitrate phenyl mercury or thiomersal. Although chelating agents can be used to improve the antioxidant and antimicrobial activity in the preparation of obestatin by specifically binding metal ions, in a preferred embodiment, the main chelating agents are: ethylenediaminetetraacetic acid (EDTA) and derivatives, and phosphoric / citric acid and salts derived.

Although the application is preferably parenteral, in a preferred embodiment, a local administration is also possible, by suitable devices to reach the tumour (i.e. endoscopic ultrasound-guided fine needle injection) and to deliver obestatin or preparations containing obestatin in the tumour or its periphery allowing a controlled release (Endoscopic ultrsound-guided injectable therapy for pancreatic cancer: a systematic review. Kaur J, et al. World J Gastroenterol. 2022; 28(21): 2383-2395. doi: 10. 3748/wjg. v28. i21.2383). In a preferred embodiment, modified-release dosage can be also used in the present invention with the intention to deliver obestatin with a delay after its administration (delayed-release dosage) or for a prolonged period of time (extended-release dosage) or to a specific target in the body (targeted-release dosage). This type of administration is focused on eliminating the limitations of the parenteral administration, and is designed to generate customizable release profiles. The implantable controlled release systems are administered by subcutaneous, intramuscular, intravenous, intradermal injection or by surgical operation. This management model, although it is more complex than the traditional administration routes, allows improving the control of the administration speed, optimizing the dose-response-duration of action, avoiding direct medical monitoring and improving the acceptance of treatment by the patient. The implants are composed of solid forms placed by minor surgery, allowing the release of the peptide continuously for prolonged periods of time. These devices are generally composed of biodegradable and biocompatible excipients, although non-biodegradable compounds can be also used (Wolinsky JB, et al. Local drug delivery strategies for cancer treatment: gels, nanoparticles, polymeric films, rods, and wafers. J Control Release. 2012; 159(1): 14-26. doi: 10.1016/j.jconrel.2011.11.031. 2. Bazeed AY et al. Pancreatic cancer:Challenges and opportunities in locoregional therapies. Cancers 2022;14:4257. Doi: 10.3390/cancers14174257).

Local drug delivery systems can be divided into two groups based on their mode of administration and mechanism of action. The first relies on systemic delivery and consists of nano-materials such as polymer nanoparticles, liposomes, and dendrimers. These delivery vehicles find their target by localization to solid tumors by passive diffusion via leaky tumor vasculature, active targeting by conjugation to a chemical moiety with an affinity for an overexpressed/unique tumor cell marker (i.e. folic acid receptor, monoclonal antibody, etc), or by triggering the release of payload from an environment-responsive nano-carrier using a local stimulus (i.e. pH, temperature, etc). These nano-materials are predominantly intended for intravenous administration.

The second group of polymer delivery vehicles includes controlled release drug delivery depot systems for implantation intra-tumorally or adjacent to the cancerous tissue. These technologies have been embodied in a variety of form-factors such as drug-eluting films, gels, wafers, rods, and particles and feature predictable and prolonged drug release kinetics. The majority of these devices are biodegradable so as to circumvent a second surgery for device removal and to avoid a chronic foreign-body immune response. The polymers used in these systems can be broadly divided in to natural and synthetic materials. Natural polymers that have been investigated for drug delivery applications include polysaccharides such as alginate, hyaluronic acid, dextran and chitosan and polypeptides including collagen, albumin, elastin, and gelatin. These materials are tolerated well in vivo, are available in abundance in nature, and can form hydrogels via self-assembly or by cross-linking. Furthermore, the property of spontaneous hydrogel formation of some natural polymers has been exploited to develop smart delivery vehicles that can be injected locally as a liquid, and upon exposure to changes in environment such as temperature, pH, or ionic composition, solidify into a hydrogel drug depot. Moreover, although the present invention is mainly directed to obestatin derived from *Homo sapiens,* it is important to note that the present invention also refers to obestatin isolated from other species which have at least 90% of identity with the obestatin derived from *Homo sapiens.* Please see Table 2 below wherein 20 aminoacid sequences that are homologous to the human obestatin have been selected by means of a BlastN analysis. The sequences were aligned using ClustalW.

**Table 2**

| *Specie | Obestatin sequence C-t (position 1) → N-t (position 23) | SEQ ID NO |
|---|---|---|
| 1 | FNAPFDVGIKLSGVQYQQHSQAL | SEQ ID NO: 1 |
| 2 | FNAPFDVGIKLSGAQYHQHSQAL | SEQ ID NO: 2 |
| 3 | FNAPFDVGIKLSGAQYQEHGQAL | SEQ ID NO: 3 |
| 4 | FNAPFDVGIKLSGAQYQQHGRAL | SEQ ID NO: 4 |
| 5 | FNAPFDVGIKLSGPQYHQHGQAL | SEQ ID NO: 5 |
| 6 | FNAPFDVGIKLSGAQYHQHGQAL | SEQ ID NO: 6 |
| 7 | FNAPFDIGIKLSGAQSLQHGQTL | SEQ ID NO: 7 |
| 8 | FNAPCDVGIKLSGAQSDQHGQPL | SEQ ID NO: 8 |
| 9 | FNAPFNIGIKLSGAQSLQHGQTL | SEQ ID NO: 9 |
| *1) Human, green monkey, drill, Papion, macaque, orangutan, gorilla, gibbon, bonobo and chimpanzee, 2) horse. 3) giant panda, 4) golden hamster, mouse and rat, 5) dog, 6) cat, 7) deer, 8) pig and 9) goat. | | |

Moreover, since it has been previously demonstrated that (11-23)-obestatin may have a biological activity analogous to obestatin, the present invention also refers to truncated obestatin, thus comprising any fragment of the original obestatin where an N- or C-terminal portion of the protein has been eliminated. Thus, in a preferred embodiment, the present invention refers to an obestatin truncated fragment of 13 amino acids: obestatin-(11-23), preferably to the truncated fragments which are included in **Table 3.**

**Table 3**

| Specie | Truncated obestatin sequence C-t (position 11) → N-t (position 23) | SEQ ID NO |
|---|---|---|
| 1 | LSGVQYQQHSQAL | SEQ ID NO: 10 |
| 2 | LSGAQYHQHSQAL | SEQ ID NO: 11 |
| 3 | LSGAQYQEHGQAL | SEQ ID NO: 12 |
| 4 | LSGAQYQQHGRAL | SEQ ID NO: 13 |
| 5 | LSGPQYHQHGQAL | SEQ ID NO: 14 |
| 6 | LSGAQYHQHGQAL | SEQ ID NO: 15 |
| 7 | LSGAQSLQHGQTL | SEQ ID NO: 16 |
| 8 | LSGAQSDQHGQPL | SEQ ID NO: 17 |
| 9 | LSGAQSLQHGQTL | SEQ ID NO: 18 |
| *1) Human, green monkey, drill, Papion, macaque, orangutan, gorilla, gibbon, bonobo and chimpanzee, 2) horse. 3) giant panda, 4) golden hamster, mouse and rat, 5) dog, 6) cat, 7) deer, 8) pig and 9) goat. | | |

Thus, departing from the sequence alignment made by using ClustalW, the inventors of the present invention designed the following Formula I for the obestatin of 23 aminoacids claimed in the present invention:

$$C\text{-FNAP}X_1X_2X_3\text{GIKLSG}X_4QX_5X_6X_7HX_8X_9X_{10}L\text{-}N \qquad \textbf{Formula I}$$

Wherein:

- $X_1$ can be substituted by F or C.
- $X_2$ can be substituted by D or N.
- $X_3$ can be substituted by V or I.
- $X_4$ can be substituted by V, A or P.
- $X_5$ can be substituted by Y or S.
- $X_6$ can be substituted by Q, H, L or D.
- $X_7$ can be substituted by Q or E.
- $X_8$ can be substituted by S or G.
- $X_9$ can be substituted by Q or R.
- $X_{10}$ can be substituted by A, T or P.

Also, departing from the sequence alignment made by using ClustalW, the inventors of the present invention designed the following Formula II of the truncated obestatin of 13 aminoacids claimed in the present invention:

$$C\text{-LSG}X_4QX_5X_6X_7HX_8X_9X_{10}L\text{-}N \qquad \textbf{Formula II}$$

Wherein:

- $X_4$ can be substituted by V, A or P.
- $X_5$ can be substituted by Y or S.
- $X_6$ can be substituted by Q, H, L or D.
- $X_7$ can be substituted by Q or E.
- $X_8$ can be substituted by S or G.
- $X_9$ can be substituted by Q or R.
- $X_{10}$ can be substituted by A, T or P.

For the purpose of the present invention the following terms are defined:

- "Pharmaceutically acceptable excipient or carrier" refers to an excipient that may optionally be included in the composition of the invention and that causes no significant adverse toxicological effects to the patient.

- By "therapeutically effective dose or amount" of obestatin, the present invention refers to the situation when obestatin is administered as described above, and brings about a positive therapeutic response in a subject having pancreatic cancer. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.

**Brief description of the figures and tables**

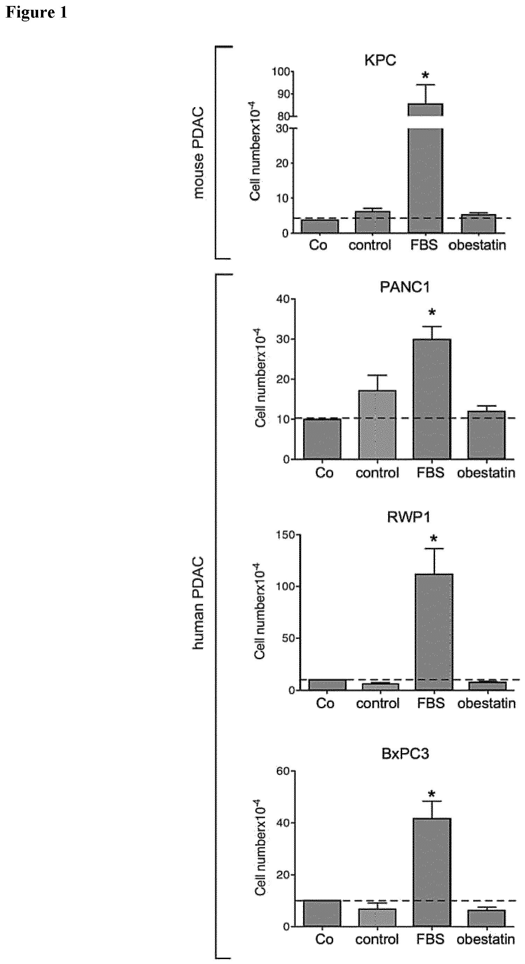

Figure 1. Proliferation assays in mice pancreatic cancer cells (KPC cells FC1242) and in human pancreatic cancer cells (PANC-1, RWP1, and BxPC3 cells) under chronic treatment of obestatin. Co: initial seeded cells; control: number of cells after cell culture for 72 h; FBS (foetal bovine serum): number of cells after FBS treatment [10% (v/v)/24h, 72h]; OB: number of cells after obestatin treatment (100 nM/24h, 72h). Results are expressed as mean$\pm$SE (n=9/group; *, P<0.05).

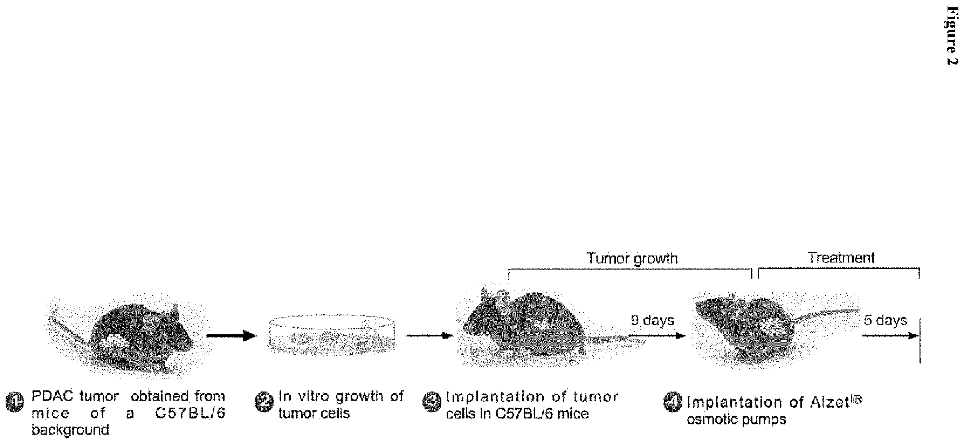

Figure 2. Schematic representation of an orthotopic syngeneic mice model established for the determination of obestatin action on tumoral development. In brief, KPC FC1242 (1$\times$10E6) cells were injected in the pancreas of C57BL/6J mice. After nine days of tumoral growing, the mice were randomly assigned to the different groups (n=4/group) and treated with vehicle [NaCl, 0.9% (w/v)] or obestatin (500 nmol/Kg/72h) via ALZET® osmotic micropumps for five days. Mice without tumoral cells treatment were utilized as healthy controls.

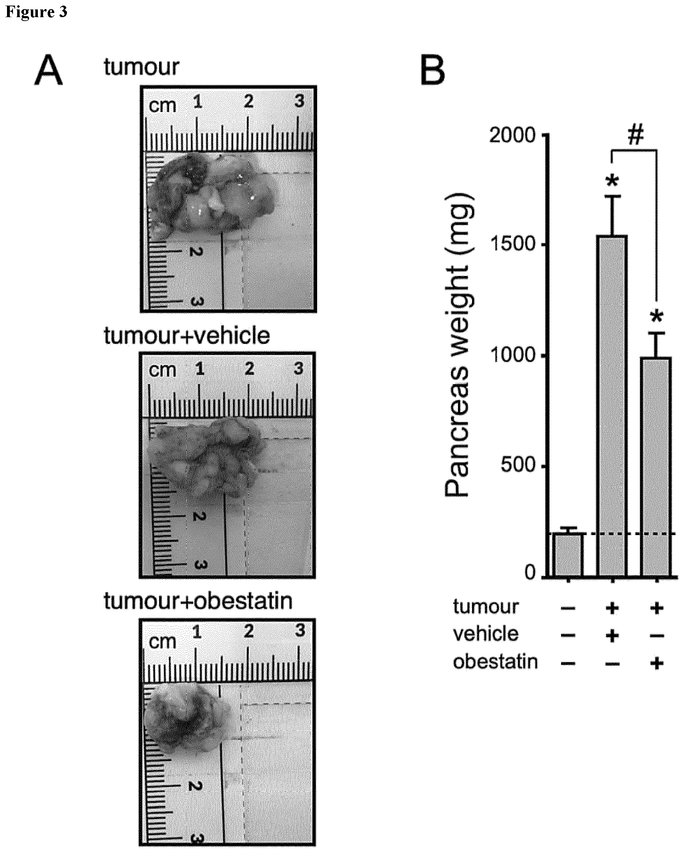

Figure 3. Effect of obestatin treatment on tumoral growing. The KPC FC1242 cells (1$\times$10E6) were injected into the pancreas of C57BL/6J mice. After nine days of tumour post-injection, mice were treated with vehicle [NaCl, 0.9% (w/v)] or obestatin (500 nmol/Kg/72h) via ALZET® osmotic micropumps for five days. Mice without tumoral cells treatment were utilized as healthy controls (n=4). Pancreases/tumours were collected, measured and weighted at day 14 after tumour implantation. Results are expressed as mean mean$\pm$SE (*, #, P<0.05).

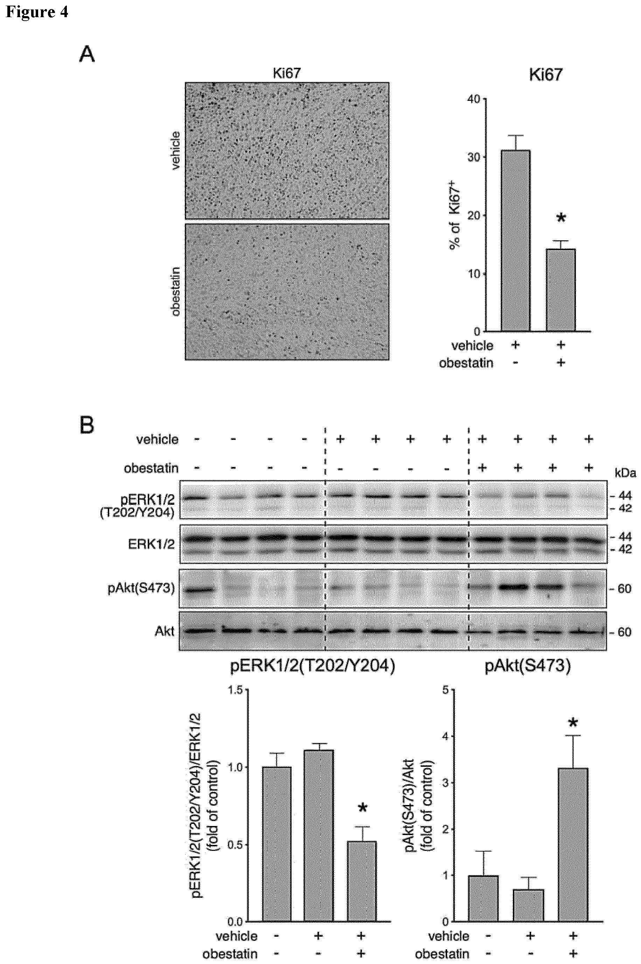

Figure 4. (A) Analysis and quantification of Ki67 positive proliferative cells by immunohistochemistry in the pancreatic tumors of C57BL/6J mice (n=4) orthotopically injected with KPC FC1242 cells and treated with vehicle [NaCl, 0.9% (w/v)] or obestatin (500 nmol/Kg/72h). Magnification: 4x. (B) Immunoblot analysis of pERK1/2(T202/Y204), ERK1/2, pAkt(S473), and Akt in tumoral pancreatic tissue. Results are expressed as fold of control of the non-treated injected mice [control; mean$\pm$SE (*, P<0.05)].

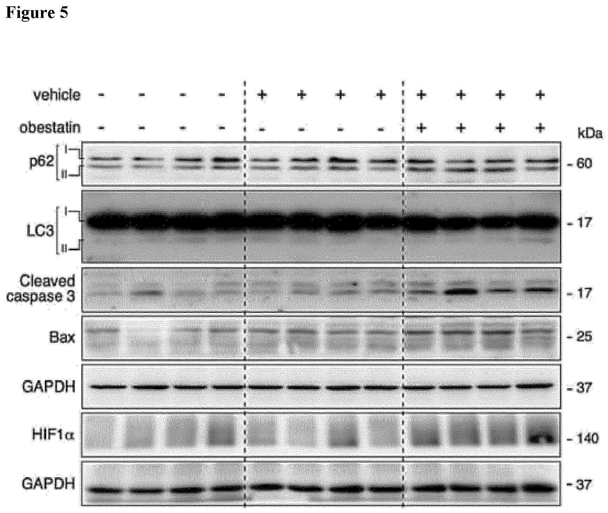

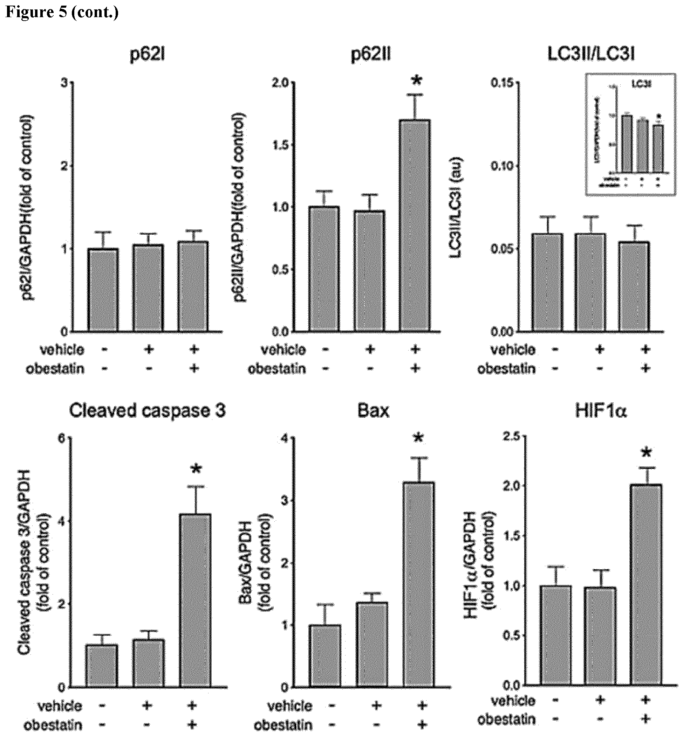

Figure 5. Immunoblot analysis of p62, LC3, cleaved caspase 3, Bax, and HIF1a in the pancreatic tumors of C57BL/6J mice (n=4) orthotopically injected with KPC FC1242 cells and treated with vehicle [NaCl, 0.9% (w/v)] or obestatin (500 nmol/Kg/72h). Results are expressed as fold of control of the non-treated injected mice [control; mean$\pm$SE (*, P<0.05)].

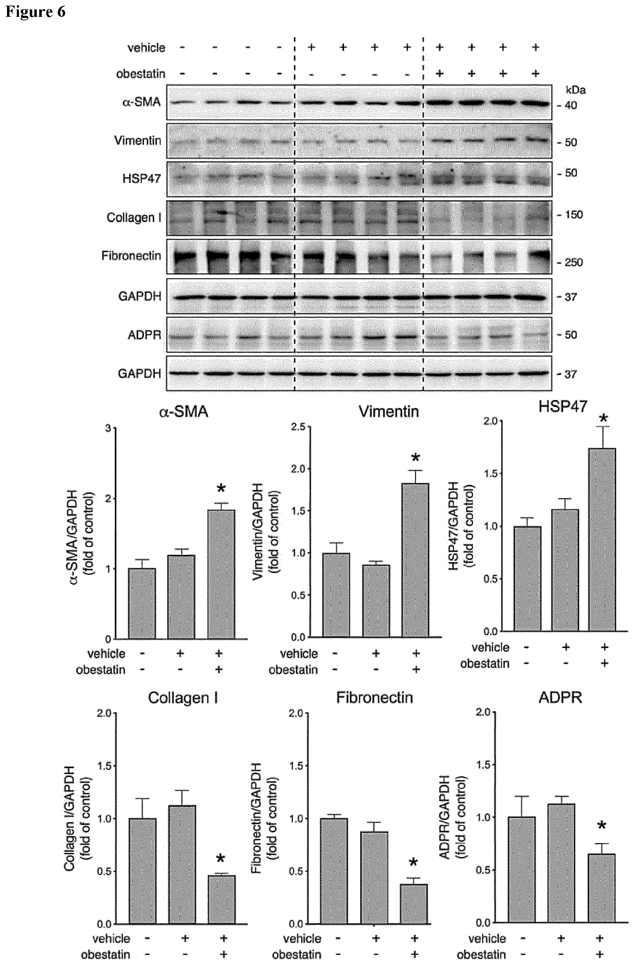

Figure 6. Immunoblot analysis of aSMA, vimentin, collagen I, fibronectin, and ADPR in the pancreatic tumors of C57BL/6J mice (n=4) orthotopically injected with KPC FC1242 cells and treated with vehicle [NaCl, 0.9% (w/v)] or obestatin (500 nmol/Kg/72h). Results are expressed as fold of control of the non-treated injected mice [control; mean$\pm$SE (*, P<0.05)].

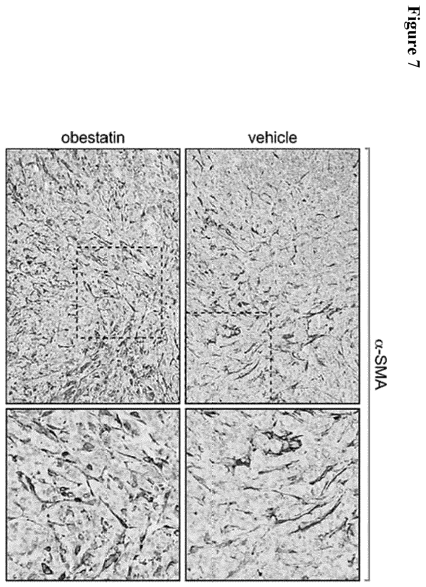

Figure 7. Immunohistochemical analysis of aSMA, vimentin, collagen I, fibronectin, and ADPR in the pancreatic tumors of C57BL/6J mice (n=4) orthotopically injected with KPC FC1242 cells and treated with vehicle [NaCl, 0.9% (w/v)] or obestatin (500 nmol/Kg/72h).

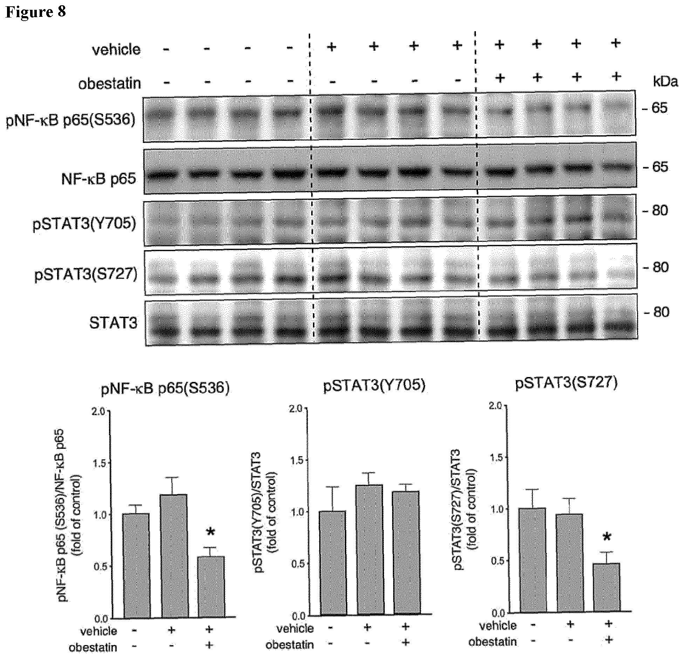

Figure 8. Immunoblot analysis of pNF-kB p65(S536), NF-kB p65, pSTAT3(Y705), pSTAT3(S727), and STAT3 in the pancreatic tumors of C57BL/6J mice (n=4) orthotopically injected with KPC FC1242 cells and treated with vehicle [NaCl, 0.9% (w/v)] or obestatin (500 nmol/Kg/72h). Results are expressed as fold of control of the non-treated injected mice [control; mean±SE (*, P<0.05)].

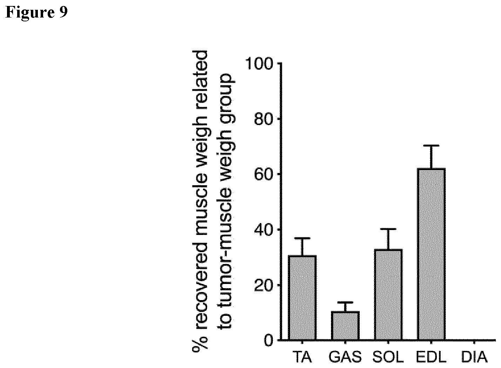

**Figure 9.** Analysis of obestatin action on skeletal muscle cachexia of of C57BL/6J mice (n=4) orthotopically injected with KPC FC1242 cells and treated with vehicle [NaCl, 0.9% (w/v)] or obestatin (500 nmol/Kg/72h). Tibialis anterior (TA), gastrocnemius (GA), soleus (SOL), extensor digitorum longus (EDL) and diaphragm (DIA) muscles were collected and weighted at day 12 after tumour injection. Results are expressed as the percentage of the weight recovery of the muscle of obestatin-treated mice related to vehicle treated mice (mean±SE).

**Detailed description of the invention**

The present invention is illustrated by means of the Examples set below without the intention of limiting its scope of protection.

**EXAMPLE 1. Material and Methods**

**Example 1.1. Cell culture**

**• KPC FC1242 cells**

The murine pancreatic cancer cells KPC FC1242 were isolated from PDAC tumour tissues obtained from LSL-KrasG12D; LSL-Trp53R172H; Pdx1-Cre mice of a pure C57BL/6 background. This cell line was kindly donated by David A. Tuveson (Cold Spring Harbor Laboratory, Cold Spring Harbor, New York, US). KPC FC1242 were maintained in DMEN (Lonza, Basel, CH), and supplemented with 10% fetal bovine serum (FBS; Hyclone, GE Healthcare Life Sciences, South Logan, UT, US), 4.5 g/L glucose, 200 mg/L sodium pyruvate, 584 mg/L L-glutamine and 1% penicillin/streptomycin (Sigma-Aldrich, St. Louis, MO, US) and incubated in an atmosphere containing 5% $CO_2$ at 37 °C. These cells were periodically tested and resulted negative for mycoplasma contamination.

**• PANC1, RWP1 and BxPC3 cells**

PANC1 cell line was obtained from ECACC (87092802; ECACC, Wiltshire, UK). RWP1 and BxPC3 human pancreatic cancer cell lines were kindly provided by FX Real (Epithelial Carcinogenesis Group, Cancer Cell Biology Programme, Spanish National Cancer Research Centre-CNIO, Madrid, ES). In brief, PANC1 cells were cultured in DMEM (supplemented with 4.5 g/L glucose, 10% FBS, and 1% penicillin/streptomycin), RWP1 and BxPC3 cell lines were maintained in RPMI (supplemented with 10% FBS and 1% penicillin/streptomycin) and incubated in an atmosphere containing 5% $CO_2$ at 37 °C. These cells were periodically tested and resulted negative for mycoplasma contamination.

**Example 1.2. Proliferation**

For the analysis of proliferation the cells were seeded on 12-well plates at an amount of 50,000 cells per well and maintained for 24 h in culture media (see above) to allow attachment. The medium was completely removed and replaced with serum-free media for additional 24 h. Then, cells were treated or not with different doses of obestatin: mouse obestatin for KPC FC1242 and human obestatin for PANC1, RWP1 and BxPC3. The following treatments were used: 0% FBS as negative control, 10% FBS as positive control, 100 nM obestatin, 200 nM obestatin, 100 nM obestatin + 10% FBS, and 200 nM obestatin + 10% FBS. These treatments were administered every 24h for three days. Twenty-four h after the last treatment, the cells were trypsinized and manually counted with a Neubauer camera.

The total cells were calculated using the following equations counting 10 squares:

$$\text{Cells per mL} = \text{the average count per square x dilution factor x 10E4}$$

$$\text{Total cells} = \text{cells per mL x the original volume of fluid from which cell sample was removed}$$

In all cases, each experiment point was replicated eight times.

**Example 1.3. Animals: the orthotopic syngeneic mice model**

*In vivo* experiments were carried out in C57BL/6 mice, obtained from the Central Animal Facility of the University of Santiago de Compostela. Male C57BL/6 mice, weighing 25 g on arrival, were housed four per cage at a constant temperature of 22±2°C and at a 12 h light/dark cycle. The mice were kept for 5 days for acclimation, with free access to water, and a standard mice chow with a composition of 5.5 % fat, 23 % protein and 70 % carbohydrate (SAFE-Panlab, Spain).

All protocols and experiments were carried out in accordance with the recommendations of the guide for the care and use of laboratory animals of the Animal Committee at the University of Santiago de Compostela. The corresponding approval of the protocol has been obtained from the Ethics Committee for Animal Experimentation University of Santiago de Compostela (15010/17/005). Experiments were performed in agreement with the rules of laboratory animal care and the international law on animal experimentation, and all efforts were made to minimize animal suffering.

**• Day 0: KPC FC1242 cells**

The cells were resuscitated and maintained in DMEN (Lonza, Basel, CH), and supplemented with 10% fetal bovine serum (FBS; Hyclone, GE Healthcare Life Sciences, South Logan, UT, US), 4.5 g/L glucose, 200 mg/L sodium pyruvate, 584 mg/L L-glutamine and 1% penicillin/streptomycin (Sigma-Aldrich, St. Louis, MO, US) and incubated in an atmosphere containing 5% $CO_2$ at 37 °C. The cells should be subcultured at least four times before implantation in mice pancreases and with a 70% of confluence, to ensure the exponential growing phase and, therefore, viability of the procedure.

**• Day 1: Implantation of KPC FC1242 cells into mouse pancreas**

**1. KPC FC1242 cells.** The cells were trypsinized with 0.05% trypsin-EDTA solution (0.5% stock trypsin-EDTA diluted 1:10 in Hanks'-based enzyme-free cell dissociation buffer), centrifuged (1,500 rpm, 5 min, 4 °C). The supernatant was removed and the pellet was washed with phosphate buffered saline (PBS, 2x). The cells were resuspended in PBS (1 mL) and transferred to a 1.5 mL-eppendorf. The cells were centrifuged (1,500 rpm, 5 min, 4 °C), the supernatant was discarded and the cells (1 × 10E6) were resuspended in Corning® Matrigel® Matrix 354230 (30 μL; Corning Inc., Corning, NY, US). The cells were kept cold, on ice, until ready to inject.

**2. Pre-surgical preparation of mice.** The cages containing the experimental mice were placed on a warmer set to 37 °C. The oxygen tank was set at a flow rate of 0.5 mL and the anaesthesia machine was set with the isofluorane vaporizer to 2-2.5%. Each single mouse was placed into the induction chamber. The mouse was monitored until no longer mobile and breathing has slowed. The mouse was quickly moved from the induction chamber and laid in ventral recumbency on the warm plate (Low Voltage Pet Heat Pad, Pet Remedy Ltd., Devon, UK). The level of induction was tested by observing any reflexive response to toe pinching. The mouse was weighed and analgesia (0.3 mg/mL buprenorphine - Bupaq®, 0.05 mg/kg) was applied subcutaneously. A small amount of eye lubricant was placed on both eyes to prevent tissue dehydration. Then, the mouse was ready for hair removal.

**3. Surgery and injection of KPC FC1242 cells.** The mouse was turned so it laid in dorsal recumbency. Then, the upper left quadrant of the abdomen was shaved and the skin was prepared with povidone-iodine followed with 70% ethanol. A longitudinal incision in the skin of the left cranial quadrant of the abdomen was made, and then the abdomen was entered by incising the muscular layer between forceps.

A 29G insulin syringe was loaded with 30 μL of cell suspension (this corresponds to 1 × 10E6 KPC FC1242 cells per injectate).

Then, the spleen and the pancreatic tail were exteriorized through the opening and the pancreatic tail was exposed for injection. Using a pair of forceps, the tail of the pancreas was grasped and a lateral tension was gently placed on it. The ventral peritoneal surface was punctured with the needle at a shallow angle, using the forceps for guidance and support. Then, the tail was released and the needle was clamped with the forceps, allowing pressure to be applied to the embolus. Thus, the cell suspension was injected into the pancreas in a slow and controlled fashion (over 10-15 s) with the syringe. During the injection process, attention was paid to possible leakage, both around the injection site (from reflux) and on the other side of the pancreatic lobule (in case of through- and-through penetration). To avoid possible leakage, the pancreas was also clamped. After injection, the needle was hold in place for 10 seconds before withdrawing to minimize leakage. A povidone-iodine-soaked swab was used to carefully dab the site to absorb any inadvertently leaked cell suspension. The spleen and the pancreas were replaced and the abdominal wall was closed with non- absorbable suture 6-0 (Ethicon, Ref: W529H) with mattress stitches, which is less accessible for mice. The mouse was monitored for about 20 min in a warmed cage until recovered from the anaesthetic. Once awake and alert, move the mouse back to its cage.

### • Day 9. Preparation of micropumps

This process was carried out in sterile conditions. Mouse obestatin was weighed and dissolved in saline. The micropump (Micro-Osmotic Pump Model 1002, Alzet®) was charged with obestatin solution to a final 500 nmol/kg72h. Each batch of Alzet® micropumps has a specific mean fill volume and a specific mean pumping rate, so these values should be taking into account for calculations. First, each empty micropump was weighed with its lid. The provided canula was used to load the micropumps. Obestatin solution, loaded in a 1 mL-syringe, was slowly transferred to the micropump. Each micropump has a volume of 100 $\mu$L approximately. It is important to avoid bubbles formation. When filled, the lid was placed on. Then, the micropump was weighed again to check correct filling. The prepared micropump was then placed in a 12-well plate, filled with saline, and then placed at 37 °C overnight.

### • Day 10. Micropumps subcutaneous implantation

The contents of the pump will be delivered into the local subcutaneous space. Absorption of the compound by local capillaries results in systemic administration. Each mouse was anesthetized as described above. Then, the mouse was laid in ventral recumbency on the warm plate. The midscapular region of the back of the animal was shaved and the skin was prepared with povidone-iodine followed with 70% ethanol for surgery. A longitudinal suitable incision was made in the skin. Using a hemostat into the incision, the subcutaneous tissue was spread to create a pocket for the micropump, approximately 1 cm longer than the micropump. The pump should not rest immediately beneath the incision, which could interfere with the healing of the incision. The filled micropump was inserted into the pocket, delivery portal first. This minimizes interaction between the compound delivered and the healing of the incision. The wound was closed with non-absorbable suture 6-0 (Ethicon, Ref: W529H) with simple stitches. The mouse was monitored for about 20 min in a warmed cage until recovered from the anaesthetic. Once awake and alert, the mouse was moved back to its cage.

The mice were assigned to the different groups (n=4/group) and treated for five days with: 1) healthy control, 2) tumour + vehicle [NaCl, 0.9% (w/v)], and 3) tumour + obestatin (500 nmol/Kg/72h). Mice without tumoral cells treatment were utilized as healthy controls.

### • Day 15. Animal slaughter

Mice were sacrificed by injectable anaesthetic overdose. The association between the injectable dissociative agent (100 mg/mL ketamine hydrochloride - Ketamidor®) and $\alpha$2-adrenergic receptor agonists (2% xylazine hydrochloride - Xylasol®) was used.

### Example 1.4. Tissue extraction and sample processing

Mice were weighed at set times over the 15-day period. The sterilized surgical material was used for the tissue extraction. Pancreases and muscles were carefully removed from each animal minimizing contamination with surrounding tissue or mouse hair. Pancreases and muscles were weighed. The skeletal muscles selected were: tibialis anterior, TA; extensor digitorum longus, EDL; gastrocnemius, GAST; soleus, SOL; and diaphragm, DIA. Pancreases were also measured with a scale (cm) and pictures were taken.

Histology - Pancreas samples for histology were fixed in 4% paraformaldehyde in PBS at room temperature overnight. After fixation, samples were dehydrated and embedded in paraffin blocks. For this study, 3-micron sections were cut from each block and placed on pre-cleaned slides (Thermo Fisher Scientific, Waltham, MA, USA). The sectioned tissue slides were covered with a thin layer of paraffin, stoked at 60°C for 1 hour and stored protected from light at room temperature to preserve the antigenicity of sections.

Immunoblot - For immunoblot analysis, pancreases were immediately frozen in dry ice and stored at -80 °C until use. Tissues were homogenized using a TissueLyser II tissue homogenizer (Qiagen, Hilden, DE), in 200 $\mu$L of RIPA lysis buffer at 30 Hz for 3 min. The lysates were clarified by centrifugation (18,000 $\times$ g, 15 min, 4 °C), and the protein concentration was quantified by the QuantiproTM BCA assay kit (Sigma-Aldrich, St. Louis, MO, US).

EP 4 434 579 A1

**Example 1.5. Histological analysis of pancreases**

The samples were immersion-fixed in 10% buffered formalin for 24 h, dehydrated and embedded in paraffin by a standard procedure. The 3 $\mu$m-thick sections were mounted on Polysine™ adhesion microscope slides (Epredia, Gerhard Menzel, Braunschweig, DE), dewaxed and rehydrated. Antigen retrieval was performed in PT-Link (Dako Agilent Technologies, Glostrup, DK) for 20 min at 97 °C in high pH buffered solution (Dako Agilent Technologies, Glostrup, DK). The immunohistochemical technique was automatically performed using an AutostainerLink 48 instrument (Dako Agilent Technologies, Glostrup, DK). The corresponding Ki-67 antibody was incubated for 30 min **(Table 1).**

**Table 1.** List of primary and secondary antibodies used in this work.

| Primary Antibodies | Use | Dilution | Code | Manufacturer |
|---|---|---|---|---|
| ADRP | IB | 1:200 | sc-377429 | Santa Cruz Biotech. |
| Akt1/2/3 | IB | 1:1000 | sc-8312 | Santa Cruz Biotech. |
| p-Akt (S473) | IB | 1:1000 | 9271 | Cell Signaling Tech. |
| Bax | IB | 1:200 | sc-20067 | Santa Cruz Biotech. |
| Cleaved caspase 3 | IB | 1:1000 | 9662 | Cell Signaling Tech. |
| Collagen I | IB | 1:1000 | ab21286 | Abcam |
| Erk1/2 | IB | 1:2000 | 9102 | Cell Signaling Tech. |
| p-Erk1/2 (T202/Y204) | IB | 1:2000 | 9101 | Cell Signaling Tech. |
| Fibronectin | IB | 1:5000 | A0245 | Dako Agilent |
| GAPDH | IB | 1:2000 | sc-32233 | Santa Cruz Biotech. |
| HIF1$\alpha$ | IB | 1:1000 | 14179 | Cell Signaling Tech. |
| HSP47 | IB | 1:200 | sc-5293 | Santa Cruz Biotech. |
| Ki-67 (SP6) | IHC | Prediluted | 275R | Sigma-Aldrich/Merck |
| LC3 A/B | IB | 1:1000 | 12741 | Cell Signaling Tech. |
| NF-$\kappa$B p65 | IB | 1:1000 | 8242 | Cell Signaling Tech. |
| p-NF-$\kappa$B p65 (S536) | IB | 1:1000 | 3033 | Cell Signaling Tech. |
| P62 | IB | 1:1000 | 5114 | Cell Signaling Tech. |
| $\alpha$-SMA | IHC IB | RTU | IR611 | Dako Agilent |
| STAT3 | IB | 1:200 | sc-8019 | Santa Cruz Biotech. |
| p-STAT3 (S727) | IB | 1:200 | sc-293059 | Santa Cruz Biotech. |
| p-STAT3 (Y705) | IB | 1:200 | sc-8059 | Santa Cruz Biotech. |
| Vimentin | IB | RTU | IR630 | Dako Agilent |
| Secondary Antibodies | Use | Dilution | Code | Manufacter |
| En Vision™ FLEX, High pH (Link) | IHC | RTU | K8000 | Dako Agilent |
| Peroxidase-AffiniPure Goat Anti-Rabbit IgG (H+L) | IB | 1:10000 | 111-035-003 | Jackson ImmunoResearch Europe |
| Peroxidase-AffiniPure Goat Anti-Mouse IgG (H+L) | IB | 1:10000 | 15-035-003 | Jackson ImmunoResearch Europe |
| RTU: ready to use; IB: immunoblot; IH: immunohistochemistry. | | | | |

EnVision™ peroxidase FLEX/HRP (Dako Agilent Technologies, Glostrup, DK) was employed as a detection system. All sections were counterstained with Harris' Hematoxylin solution (HHS) for 15 min. For $\alpha$-SMA detection, the immunohistochemical protocol has been performed manually with the following modifications: 1) epitope retrieval in high pH buffered solution using a PT-Link (Dako Agilent Technologies, Glostrup, DK) for 20 min at 97 °C); 2) incubation with peroxidase-blocking agent (5 min); 3) incubation with primary antibody in EnVision FLEX Antibody Diluent (20 min, room temperature; **Table 1);** 4) incubation with HRP (20 min); 5) incubation with DAB-tetrahydrochloride (10 min); and 6) counterstaining with HHS (15 min). Photographs were taken using Olympus BX51 microscope (Olympus Corporation, Tokyo, JP).

- **Ki67 analysis.** Ki67 was analysed immunohistochemically. Photographs were taken using a Zeiss Observer Z1 microscope and the Axiovision software (carl Zeiss, Göttingen, DE). For Ki67 expression quantification in the samples, ten fields of the tumour representative DAB images were quantified by using the Fiji/Image J software.

**Example 1.6. Immunoblot analysis of pancreases**

Tissues were disrupted and homogenized in ice-cold lysis buffer [50 mM Tris-HCl pH 7.2, 150 mM NaCl, 1 mM EDTA, 1% (v/v) NP-40, 0.25% (w/v) Na-deoxycholate, protease inhibitor cocktail (PIC; 1:100; Sigma Chemical Co., St. Luis, MO, US), phosphatase inhibitor cocktail (PHIC; 1: 100; Sigma Chemical Co., St. Luis, MO, US)] with 5 mm stainless steel beads using a TissueLyser (Qiagen, Hilden, DE) at 30 Hz for 3 min. The soluble cell lysates were precleared by centrifuging at 14,000 rpm for 15 min. Supernatants were recovered and the protein was quantified with BCA Protein Assay Kit (Thermo Fisher Scientific, Rockford, IL, US). The same amount of protein for each sample was separated on 7-15% SDS/polyacrylamide gels and transferred to nitrocellulose membranes (Bio-Rad, Hercules, CA, US). The blots were incubated with 5% bovine serum albumin (BSA) in a Tris buffer solution (TBS) containing Tween-20 [TBST; 20 mM Tris-HCl (pH 8.0), 150 mM NaCl, 0.1% (v/v) Tween-20, solution used for all incubation and washing steps] for 1 h. The blots were then incubated with the corresponding primary antibodies and subsequently were incubated with the corresponding peroxidase-conjugated IgG antibody (**Table 1).** After washing, the signals were visualized using an ECL plus Western Blotting Detection System (Pierce ECL Western Blotting Substrate; Thermo Fisher Scientific, Pierce, Rockford, IL, US). The image processing was performed using the NIH Image Software ImageJ 1.53o (National Institutes of Health, Bethesda, MD, US).

**Example 1.7. Data analysis**

All the data were reported as the mean $\pm$ standard error of the mean (SEM). ROUT test with a Q=5% was performed to statistically detect the presence of outliers. Kolmogorov-Smirnov test with Dallal-Wikilnson-Lilief P value was performed to evaluate the normality of each data set. Unpaired Student t-test was used to compare means for data sets with a normal distribution. Mann-Whitney test was used to compare medians for data sets with non-normal distribution. Values of $P<0.05$ were considered to be statistically significant and are marked with an asterisk (*). Asterisk (**, ***, ****) denotes $P<0.01$, $P<0.001$ and $P<0.0001$, respectively.

**EXAMPLE 2. Results**

**Example 2.1. Obestatin does not stimulate proliferation in pancreatic cancer cells**

**Example 2.1** shows that obestatin does not exert mitogenic features in pancreatic cancer (PC) cells both murine (KPC cells) and human (PANC1, RWP1 and BxPC3) **Figure 1.** Indeed, obestatin chronic treatment (100 nM each 24h for 72 h) does not activate the proliferation of the different PC cell lines. FBS (10%) was used as a positive control of the proliferation. However, obestatin does exert a significant inhibitory effect on the FBS-mediated proliferation (100 nM obestatin + 10% FBS each 24h for 72 h) downregulating proliferation to initial control levels.

**Example 2.2. Obestatin inhibits pancreatic tumour growth in an in vivo model of pancreatic cancer**

This **Example 2.2** shows that obestatin treatment inhibits tumoral growth. Following the experimental scheme provided in **Figure 2,** the mice were subjected to the evaluation of obestatin action on their tumoral development. After nine days of tumoral growing, the mice were randomly assigned to the different groups (n=4/group) and treated with vehicle [NaCl, 0.9% (w/v)] or obestatin (500 nmol/Kg/72h) via ALZET® osmotic micropumps for five days. Mice without tumoral cells treatment were utilized as healthy controls. After five days of obestatin treatment, a significant reduction in the pancreatic tumour growing was observed in weight (35.8±6.9 %; **Figure 3).**

**Example 2.3. Obestatin inhibits proliferation in pancreatic tumours**

Ki-67 is a well-known marker of cellular proliferation. For the murine tissues, the MIB-5 clone was used. **Figure 4A** illustrates Ki67 different expression in the tumour area of the pancreas of mice treated or not with obestatin and shows a 56.0±5.2 % diminution in the number of Ki67 positive cells in the obestatin treated animals.

Obestatin exerts its effects mainly via kinase panels in which ERK1/2 and Akt are the key signalling nodes. The metabolic pathway controlled by MAPK (ERK1/2) is characterized mainly by its proliferative role. In parallel, the Akt controls the signalling pathway triggering effects on cell growth, proliferation and differentiation. **Figure 4B** shows the variation in the activation of pERK1/2(T202/Y204) and pAkt(S473) in tumoral pancreatic tissue in obestatin-treated, vehicle-treated compare with tumoral non treated animals. Western blotting showed that the total expressions of ERK and AKT were found to be unchanged. Each row corresponds to each animal (n=4 per group) and values are referenced to non-treated animals. Obestatin causes a reduction in ERK1/2 activation of almost 50% in obestatin-treated mice, which correlates with the observed diminution in Ki67 expression (56.0±5.2 %). On the contrary, Akt activation was augmented in obestatin-treated animals (369.5±100.1 %).

**Example 2.4. Obestatin activates apoptosis and inhibits autophagic flux in the tumour**

**Figure 5** shows the effect of obestatin in the Bad-Bax-caspase-3 cascade, the canonical apoptosis pathway. The intrinsic pathway can be initiated in mitochondria by stress stimuli and is regulated by the balance of the action of pro-apoptotic and anti-apoptotic Bcl-2 protein family members. Obestatin treatment caused an increase in levels of the proapoptotic Bax (141.6±28.4 %) and its downstream effector caspase-3 (cleaved caspase 3; 264.6±57.7 %). This proapoptotic activity was also associated to the inhibition of the autophagic flux via the regulation of LC3-II and p62. Obestatin treatment causes a diminution in LC3-I (9.2±7.5 %) and, also a reduction in LC3-II levels (21.8±7.3 %) with a significant upregulation in p62 (75.7±20.8 %). The autophagosome marker LC3B-II and p62 were accumulated in obestatin treated mice, suggesting that late-stage autophagy was blocked.

**Example 2.5. Obestatin activates the hypoxia inducible factor 1 alpha**

Pancreatic ductal adenocarcinomas are hypovascular, resulting in the up-regulation of hypoxia inducible factor 1 alpha (HIF1a), which promotes the survival of cells under low-oxygen conditions. HIF1$\alpha$ can also act as a tumour suppressor by preventing the expression of PPP1R1B and subsequent degradation of the p53 protein in pancreatic cancer cells. **Figure 5** shows that obestatin treated mice presented increased expression of HIF1$\alpha$ compared to control or vehicle-treated animals (106.6±17.8 %).

**Example 2.6. Obestatin modulates the response of the cancer associated fibroblasts**

Pancreatic cancer is characterized by abundant desmoplasia that constitutes up to 90 % of the total tumour volume and contains extracellular matrix (ECM), immune cells, vasculature, and cancer-associated fibroblasts. CAFs secrete ECM and soluble factors that stimulate cancer progression, and are believed to be derived from mesenchymal cells of different origins that are resident or recruited to the pancreas by neoplastic cells. Notably, obestatin treatment inhibited the expression of the fibrosis-related markers collagen (60.1±2.9 %) and fibronectin (57.0±7.1 %) **(Figure 6),** implying that this peptide reorganizes the tumour microenvironment towards conditions unfavourable for tumour growth through the regulation of fibrosis. A major source of CAFs in pancreatic cancer is pancreatic stellate cells (PSCs), which are resident mesenchymal cells of the pancreas that store lipid droplets and express fibroblast-activation protein $\alpha$ (FAP). Upon activation, PSCs express the myofibroblast protein $\alpha$-smooth muscle actin ($\alpha$SMA) and secrete factors that stimulate tumour growth, cell survival, and metastasis. Paradoxically, obestatin increased the expression of the myofibroblasts-CAF (my-CAF) marker, aSMA (53.7±7.3 %; **Figure 6). Figure 7** also shows the spatial distribution of $\alpha$SMA, a hallmark of myofibroblasts, in the pancreatic tumours of the obestatin treated animals. Heat shock protein 47 (HSP47) is a collagen-specific molecular chaperone that is indispensable for the proper folding and secretion of collagen into the extracellular space. The chaperone also functions as a nodal hub in the regulation of the ECM network, and it is associated to activated PSCs. Obestatin treated mice presented higher levels of this protein (49.5±17.7 %; **Figure 6).** Vimentin is a major intermediate filament protein present in mesenchymal cells that plays an important role in the development, invasion, and metastasis of cancer. Activated PSCs expressed higher levels of this mesenchymal marker, and it participates in the ability of these cells to migrate. The augment observed for vimentin in the obestatin treated tumours is shown in (111.1±19.1 %), shown in Figure 6. Moreover, adipophilin (ADRP) a stablished marker of quiescent PSCs, was diminished in obestatin treated pancreases (42.6±8.8 %; **Figure 6).**

Furthermore, obestatin treatment inhibited the NF-kB and JAK/STAT pathways, nodes involved in stromal modification and tumour growth through the determination of CAFs (inflammatory-type myCAF/CAF ratio, iCAF), by downregulating pNF-KB p65(S536) and pSTAT3(S727) in a 43.6±9.0 % and 52.3±12.4 %, respectively **(Figure 8).** Although we cannot exclude that these effects are partly a consequence of a direct attack on tumour cells, these results suggest that obestatin regulates the plasticity of CAFs towards a myCAF phenotype.

**Example 2.7. Obestatin mitigates the pancreatic cancer associated cachexia**

Cancer cachexia is a highly debilitating condition characterized by weight loss and muscle wasting that contributes significantly to the morbidity and mortality of pancreatic cancer. To study the effect of obestatin on the muscle atrophy caused by the pancreatic cancer in our mouse model, the following skeletal muscles were analysed: tibialis anterior (TA), gastrocnemius (GA), soleus (SOL), extensor digitorum longus (EDL) and diaphragm (DIA). **Figure 9** shows that obestatin treatment notably mitigated the manifestations of pancreatic cancer-associated cachexia, reducing the skeletal muscle weight loss caused by the tumour in $30.8\pm6.1$ % for TA, $10.6\pm3.2$ % for GAS, $33.0\pm7.3$ % for SOL, and $62.2\pm8.1$ % for EDL. No effect was observed in the DIA muscle. The observed differences could have relationship with fibre type composition of the selected muscles. The results are expressed as the percentage of the weight recovery of the muscle of obestatin-treated mice related to vehicle treated mice.

**Claims**

1. Obestatin consisting of the amino acid sequence of Formula I:

$$C\text{-FNAP}X_1X_2X_3\text{GIKLSG}X_4QX_5X_6X_7HX_8X_9X_{10}L\text{-}N \qquad \text{(I)}$$

or a fragment thereof consisting of the aminoacid sequence of Formula II:

$$C\text{-LSG}X_4QX_5X_6X_7HX_8X_9X_{10}L\text{-}N \qquad \text{(II)}$$

or a molecule comprising said obestatin of Formula I or fragment of Formula II,
for use in the treatment of pancreatic cancer, wherein: $X_1$ can be substituted by the amino acid F or C, $X_2$ can be substituted by the amino acid D or N, $X_3$ can be substituted by the amino acid V or I, $X_4$ can be substituted by the amino acid V, A or P, $X_5$ can be substituted by the amino acid Y or S, $X_6$ can be substituted by the amino acid Q, H, L or D, $X_7$ can be substituted by the amino acid Q or E, $X_8$ can be substituted by the amino acid S or G, $X_9$ can be substituted by the amino acid Q or R, and $X_{10}$ can be substituted by the amino acid A, T or P.

2. Obestatin for use, according to the claim 1, wherein the amino acid sequence of Formula I is selected from the list consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 or SEQ ID NO: 9 or wherein the amino acid sequence of Formula II is selected from the list consisting of: SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17 or SEQ ID NO: 18.

3. Obestatin for use, according to any of the claims 1 or 2, wherein the obestatin is administered systemically or locally.

4. Pharmaceutical composition comprising obestatin and, optionally, pharmaceutically acceptable excipients or carriers, wherein the obestatin consists of the amino acid sequence of Formula I:

$$C\text{-FNAP}X_1X_2X_3\text{GIKLSG}X_4QX_5X_6X_7HX_8X_9X_{10}L\text{-}N \qquad \text{(I)}$$

and the fragment thereof consisting of the aminoacid sequence of Formula II:

$$C\text{-LSG}X_4QX_5X_6X_7HX_8X_9X_{10}L\text{-}N \qquad \text{(II)}$$

wherein: $X_1$ can be substituted by the amino acid F or C, $X_2$ can be substituted by the amino acid D or N, $X_3$ can be substituted by the amino acid V or I, $X_4$ can be substituted by the amino acid V, A or P, $X_5$ can be substituted by the amino acid Y or S, $X_6$ can be substituted by the amino acid Q, H, L or D, $X_7$ can be substituted by the amino acid Q or E, $X_8$ can be substituted by the amino acid S or G, $X_9$ can be substituted by the amino acid Q or R, and $X_{10}$ can be substituted by the amino acid A, T or P.

5. Pharmaceutical composition, according to claim 4, wherein the amino acid sequence of Formula I is selected from the list consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 or SEQ ID NO: 9 or wherein the amino acid sequence of Formula II is selected from the list consisting of: SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17 or SEQ ID NO: 18.

6. Pharmaceutical composition, according to any of the claims 4 to 5, for use in the treatment of pancreatic cancer.

7. Pharmaceutical composition, according to any of the claims 4 to 6, wherein the composition is administered systemically or locally.

**Figure 1**

**Figure 2**

**Figure 3**

A tumour

tumour+vehicle

tumour+obestatin

B

**Figure 4**

**Figure 5**

**Figure 5 (cont.)**

**Figure 6**

**Figure 7**

Figure 8

**Figure 9**

Europäisches
Patentamt

European
Patent Office

Office européen
des brevets

# EUROPEAN SEARCH REPORT

Application Number

EP 23 38 2269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEAL-L OPEZ S ET AL: "e-P237 - Effect of obestatin on the migration and invasion of pancreatic tumor cells.", PANCREATOLOGY, vol. 20, 4 December 2020 (2020-12-04), page S135, XP093064816, DOI: 10.1016/j.pan.2020.07.252 | 4,5,7 | INV. A61P5/48 A61K38/25 C07K14/60 |
| Y | * abstract * | 1-7 | |
| X | Estévez Pérez Lara Sofía: "Endogenous systems involved in the development of pancreatic cancer: role of the obestatin/GPR39 system", , 2019, XP093065129, Retrieved from the Internet: URL:https://minerva.usc.es/xmlui/bitstream /handle/10347/18839/rep_1792.pdf?sequence= 1&isAllowed=y [retrieved on 2023-07-19] | 4,5,7 | |
| Y | * the whole document * * Results * * Conclusions * | 1-7 | TECHNICAL FIELDS SEARCHED (IPC) A61K A61P C07K |
| X | WO 2020/058533 A1 (UNIV SANTIAGO COMPOSTELA [ES]; SERVIZO GALEGO DE SAUDE [ES]) 26 March 2020 (2020-03-26) | 4,5,7 | |
| Y | * the whole document * * claims; examples * | 1-7 | |
| Y | WO 2009/033795 A2 (MONDOBIOTECH LAB AG [LI]; BEVEC DORIAN [DE] ET AL.) 19 March 2009 (2009-03-19) * the whole document * * claims; examples * | 1-7 | |

The present search report has been drawn up for all claims

| Place of search | Date of completion of the search | Examiner |
|---|---|---|
| Munich | 19 July 2023 | Orlando, Michele |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 38 2269

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2023

| Patent document cited in search report | Publication date | Patent family member(s) | | Publication date |
|---|---|---|---|---|
| WO 2020058533 A1 | 26-03-2020 | NONE | | |
| WO 2009033795 A2 | 19-03-2009 | AU | 2008297875 A1 | 19-03-2009 |
| | | AU | 2008297891 A1 | 19-03-2009 |
| | | AU | 2008297898 A1 | 19-03-2009 |
| | | AU | 2008297909 A1 | 19-03-2009 |
| | | AU | 2008306217 A1 | 09-04-2009 |
| | | AU | 2008306255 A1 | 09-04-2009 |
| | | AU | 2008309993 A1 | 16-04-2009 |
| | | AU | 2008310078 A1 | 16-04-2009 |
| | | CA | 2698687 A1 | 09-04-2009 |
| | | CA | 2698778 A1 | 19-03-2009 |
| | | CA | 2698828 A1 | 19-03-2009 |
| | | CA | 2698971 A1 | 19-03-2009 |
| | | CA | 2698977 A1 | 19-03-2009 |
| | | CA | 2699084 A1 | 16-04-2009 |
| | | CA | 2699104 A1 | 09-04-2009 |
| | | CA | 2699169 A1 | 16-04-2009 |
| | | EP | 2185171 A2 | 19-05-2010 |
| | | EP | 2185177 A1 | 19-05-2010 |
| | | EP | 2187921 A2 | 26-05-2010 |
| | | EP | 2187944 A1 | 26-05-2010 |
| | | EP | 2190450 A2 | 02-06-2010 |
| | | EP | 2190456 A2 | 02-06-2010 |
| | | EP | 2190459 A1 | 02-06-2010 |
| | | EP | 2197466 A2 | 23-06-2010 |
| | | EP | 2205269 A1 | 14-07-2010 |
| | | JP | 5385279 B2 | 08-01-2014 |
| | | JP | 2010539002 A | 16-12-2010 |
| | | JP | 2010539008 A | 16-12-2010 |
| | | JP | 2010539016 A | 16-12-2010 |
| | | JP | 2010539022 A | 16-12-2010 |
| | | JP | 2010539028 A | 16-12-2010 |
| | | JP | 2010539051 A | 16-12-2010 |
| | | JP | 2010539056 A | 16-12-2010 |
| | | JP | 2010539059 A | 16-12-2010 |
| | | KR | 20100056510 A | 27-05-2010 |
| | | KR | 20100057049 A | 28-05-2010 |
| | | KR | 20100057057 A | 28-05-2010 |
| | | KR | 20100057641 A | 31-05-2010 |
| | | KR | 20100057642 A | 31-05-2010 |
| | | KR | 20100058554 A | 03-06-2010 |
| | | KR | 20100059859 A | 04-06-2010 |
| | | KR | 20100059865 A | 04-06-2010 |
| | | RU | 2010113972 A | 20-10-2011 |
| | | RU | 2010113985 A | 20-10-2011 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 23 38 2269

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2023

| Patent document cited in search report | Publication date | Patent family member(s) | | Publication date |
|---|---|---|---|---|
| | | RU | 2010114004 A | 20-10-2011 |
| | | RU | 2010114013 A | 20-10-2011 |
| | | RU | 2010114024 A | 20-10-2011 |
| | | RU | 2010114043 A | 20-10-2011 |
| | | RU | 2010114056 A | 20-10-2011 |
| | | RU | 2010114057 A | 20-10-2011 |
| | | US | 2010190717 A1 | 29-07-2010 |
| | | US | 2010204090 A1 | 12-08-2010 |
| | | US | 2010204107 A1 | 12-08-2010 |
| | | US | 2010204108 A1 | 12-08-2010 |
| | | US | 2010204109 A1 | 12-08-2010 |
| | | US | 2010204139 A1 | 12-08-2010 |
| | | US | 2010210538 A1 | 19-08-2010 |
| | | US | 2010249017 A1 | 30-09-2010 |
| | | US | 2010323950 A1 | 23-12-2010 |
| | | WO | 2009033701 A1 | 19-03-2009 |
| | | WO | 2009033707 A2 | 19-03-2009 |
| | | WO | 2009033717 A2 | 19-03-2009 |
| | | WO | 2009033724 A1 | 19-03-2009 |
| | | WO | 2009033735 A2 | 19-03-2009 |
| | | WO | 2009033744 A1 | 19-03-2009 |
| | | WO | 2009033795 A2 | 19-03-2009 |
| | | WO | 2009039990 A2 | 02-04-2009 |
| | | WO | 2009040020 A1 | 02-04-2009 |
| | | WO | 2009043439 A2 | 09-04-2009 |
| | | WO | 2009043448 A2 | 09-04-2009 |
| | | WO | 2009043450 A2 | 09-04-2009 |
| | | WO | 2009043457 A2 | 09-04-2009 |
| | | WO | 2009043458 A2 | 09-04-2009 |
| | | WO | 2009043466 A2 | 09-04-2009 |
| | | WO | 2009043505 A2 | 09-04-2009 |
| | | WO | 2009043527 A2 | 09-04-2009 |
| | | WO | 2009046844 A1 | 16-04-2009 |
| | | WO | 2009046852 A1 | 16-04-2009 |
| | | WO | 2009046861 A1 | 16-04-2009 |
| | | WO | 2009046865 A2 | 16-04-2009 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BEGOÑA O. ALÉN et al.** The NMR structure of human obestatin in membrane-like environments: insights into the structure-bioactivity relationship of obestatin. *PLoS One,* 2012, vol. 7 (10), e45434
- **DOMÍNGUEZ-MUÑOZ JE et al.** Impact of the treatment of pancreatic exocrine insufficiency on survival of patients with unresectable pancreatic cancer: a retrospective analysis. *BMC Cancer,* 2018, vol. 18 (1), 534
- **TEMPERO MA.** NCCN Guidelines Updates: Pancreatic Cancer. *J Natl Compr Canc Netw.,* 01 May 2019, vol. 17 (5.5), 603-605
- **KAUR J et al.** Endoscopic ultrsound-guided injectable therapy for pancreatic cancer: a systematic review. *World J Gastroenterol.,* 2022, vol. 28 (21), 2383-2395
- **WOLINSKY JB et al.** Local drug delivery strategies for cancer treatment: gels, nanoparticles, polymeric films, rods, and wafers. *J Control Release.,* 2012, vol. 159 (1), 14-26
- **BAZEED AY et al.** Pancreatic cancer:Challenges and opportunities in locoregional therapies. *Cancers,* 2022, vol. 14, 4257